# EUROPEAN PATENT SPECIFICATION

(11) **EP 2 521 482 B1**
(45) Date of publication and mention of the grant of the patent: **07.08.2013**
(21) Application number: 11701362.3
(22) Date of filing: 03.01.2011
(51) Int. Cl.: A61B 3/16

(54) **INTRAOCULAR PRESSURE MONITORING DEVICE**
VORRICHTUNG ZUR ÜBERWACHUNG DES AUGENINNENDRUCKS
DISPOSITIF DE SURVEILLANCE DE LA PRESSION INTRAOCULAIRE

(30) Priority: 05.01.2010 WO PCT/EP2010/050062
(43) Date of publication of application: 14.11.2012
(73) Proprietor: Sensimed SA, 1007 Lausanne (CH)
(72) Inventor: CERBONI, Sacha, CH-1007 Lausanne (CH)
(74) Representative: KATZAROV S.A.
(86) International application number: PCT/EP2011/050038
(87) International publication number: WO 2011/083105

(56) References cited:
- WO-A1-03/001991
- WO-A1-2009/049686
- US-A- 4 089 329
- LEONARDI MATTEO ET AL: "Wireless contact lens sensor for intraocular pressure monitoring: assessment on enucleated pig eyes.", June 2009 (2009-06), ACTA OPHTHALMOLOGICA JUN 2009 LNKD- PUBMED:19016660, VOL. 87, NR. 4, PAGE(S) 433 - 437, XP002600763, ISSN: 1755-3768 figure 1 chapter "Materials and Methods", in particular: page 434, middle column, line 21 - line 33 page 434, right-hand column, line 9 - line 22
- "Applying MEMS technology to the diagnosis of glaucoma", MEMS Investor Journal, Weekly Newsletter , 19 February 2009 (2009-02-19), XP002600775, Retrieved from the Internet: URL:http://www.memsinvestorjournal.com/200 9/02/applying-mems-technology-to-the-diagn osis-of-glaucoma-.html [retrieved on 2010-09-14]

## Description

The present invention relates to a device for monitoring the intraocular pressure (IOP). The present invention relates in particular to a device that can be placed on the eye of a user to monitor intraocular pressure over an extended period of time, for example 24 hours or more. The present invention also relates to a kit and to a system for monitoring the intraocular pressure (IOP).

Glaucoma is a widespread disease characterized by an elevated intraocular pressure (IOP). This elevated IOP produces a gradual loss of peripheral vision. There is therefore a need to have a detailed knowledge of IOP in glaucoma patients in order to provide reliable diagnostics or for setting up new therapies.

Patent EP1401327 describes an intraocular pressure recording system comprising a soft contact lens and a pressure sensor fixed to the contact lens. The pressure sensor comprises an active strain gage which is located around the center of the contact lens, thus allowing measuring the spherical deformations of the eyeball that are due to IOP changes. In one embodiment, the pressure sensor comprises two active strain gages and two passive strain gages placed in a Wheatstone bridge configuration. The active strain gages are circular gages situated around the center of the contact lens, while the passive strain gages are placed essentially radially to the lens in order to minimize their deformation when the eyeball is deformed. The passive strain gages are made of several radial segments located on one side of the contact lens, which are interconnected by short and substantially tangential segments.

The article "Wireless contact lens sensor for intraocular pressure monitoring: assessment on enucleated pig eyes.", Matteo Leonardi, Elie M. Pitchon, Arnaud Bertsch, Philippe Renaud and Andre Mermoud - Acta Ophtalmologica, June 2009; Vol. 87, pages 433-437, describes a similar intraocular pressure recording system with passive strain gages placed radially on the soft contact lens.

A drawback of this intraocular pressure recording system is that it is difficult to optimize the characteristics of the sensor without compromising the comfort of the user. For the passive strain gage to be as insensitive as possible to the deformations of the eyeballs, the radial segments should be as long as possible relative to the tangential segments. However their length is limited because if they reach too close to the center of the lens they lay within the sight of the user. And even if the length of the radial segments is correctly limited for a standard use of the contact lens, one can't exclude situations where the user's sight might be disturbed by the passive strain gages, for example if the contact lens accidentally only slightly slides on the eye, or in a dark environment, where the user's pupil is particularly dilated.

Another drawback of this intraocular pressure recording system is that the asymmetrical design of the passive strain gages relative to the center of the contact lens could lead to asymmetrical temporary or permanent deformations of the contact lens itself, which might then loose its spherical shape, thus resulting in discomfort for the user wearing the lens.

Still another drawback of the intraocular pressure recording system of EP1401327 is that the position and the shape of the passive strain gages are very different from those of the active strain gages. The influence of variations in environmental factors other than the IOP, for example of the temperature, the humidity, etc., on the physical properties of the passive strain gages might therefore differ significantly from the influence of the same variations on the physical properties of the active strain gages, thus inducing errors or inaccuracies when determining the IOP.

An aim of the present invention is therefore to provide an intraocular pressure monitoring device that can be worn over extended periods of time and in any situation without major discomfort for the user.

Another aim of the present invention is to provide an intraocular pressure monitoring device that delivers an accurate measurement of the IOP.

Still another aim of the present invention is to provide a kit and an intraocular pressure monitoring system that can deliver an accurate measurement of the IOP over an extend period of time.

These aims and other advantages are achieved by a device, a kit and a system comprising the features of the corresponding independent claims.

These aims are achieved in particular by an intraocular pressure monitoring device comprising a soft contact lens and a pressure sensor united with the contact lens, the pressure sensor comprising an active strain gage, a passive strain gage, a rigid element and a microprocessor. The active strain gage, passive strain gage and rigid element are placed at a distance from the center of the contact lens, the active strain gage comprising a portion encircling the center of the contact lens on at least 180°, wherein the passive strain gage and the rigid element each comprise a portion encircling the center of the contact lens on at least 180°, and wherein the portion of the passive strain gage situated around the center of the contact lens is placed in immediate vicinity of the portion of the rigid element situated around the center of the contact lens.

These aims are also achieved by a kit comprising such a pressure monitoring device and a portable recording device configured for communicating with the pressure monitoring device and for storing data received from it.

These aims are also achieved by an intraocular pressure monitoring system comprising such a kit and a computing device configured for communicating with the portable recording device for receiving and/or processing and/or storing data received from the portable recording device.

According to the invention, the intraocular pressure monitoring device comprising a rigid element for rigidifying a part of the contact lens, it allows placing the passive strain gage in vicinity of this rigid element around the center of the contact lens, thereby allowing the design of passive strain gages that do not impair the user's sight, and also allowing the design of passive strain gages with a configuration similar to that of the active gage in order to provide for a more efficient and reliable correction of the variations measured by the active gage that are due to environmental factors and not to IOP variations.

The passive strain gage being placed in immediate vicinity of the rigid element, its shape can be freely chosen without almost any constraint, because its resistance to deformations of the eyeball of a user wearing the device of the invention is provided by the rigid element rather than by its shape and/or position or orientation on the contact lens. This allows for example designing a passive strain gage situated around the center of the contact lens, which is essentially symmetrical relative to the center of the contact lens. Furthermore, the passive strain gage can be designed and positioned similarly to the active strain gage. The passive strain gage can for example be an essentially continuous conductor, for example circular or polygonal, which is at least partly situated around the center of the lens. The passive strain gage can then easily be placed at a distance from the center of the contact lens sufficient for not disturbing the user.

The present invention will be better understood with the help of the following description illustrated by the figures, where:
Fig. 1 illustrates an intraocular pressure monitoring device according to a preferred embodiment of the invention;
Fig. 2 is a cut view of the device of figure 1 along the II-II line;
Fig. 3 illustrates an intraocular pressure monitoring device according to another embodiment of the invention;
Fig. 4 shows an example of a possible configuration of a passive and/or of an active strain gage according to the invention;
Fig. 5a and 5b illustrate two possible variant embodiment for the configuration of a passive and/or of an active strain gage according to the invention;
Fig. 6 is a schematic representation of an intraocular pressure monitoring system according to the invention.

The same reference numbers in different figures designate the same or similar elements.

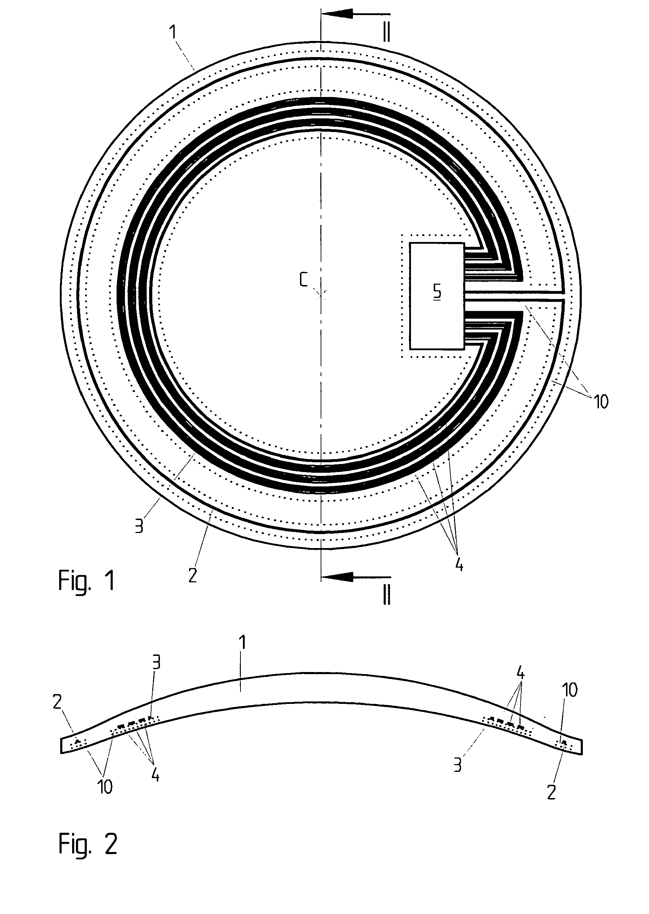

According to a preferred embodiment illustrated in Fig. 1, the intraocular monitoring device of the invention comprises a pressure sensor united with a contact lens 1, preferably a soft contact lens. When the contact lens 1 is worn by a user, the pressure sensor is placed on the eyeball of the user. In order to avoid any discomfort for the user, the elements of the pressure sensor are preferably not in direct contact with the eye. The sensor is for example incorporated, or embedded, within the contact lens 1 or affixed to the external, convex, surface of the contact lens 1, or a combination thereof, some elements of the sensor being embedded within the contact lens 1 and others being affixed on its surface.

However, according to other, less advantageous, embodiments of the invention, part or all elements of the pressure sensor are affixed on the inner, concave, surface of the contact lens 1 and are thus at least partly in direct contact with the eye of the user wearing the contact lens 1.

The elements of the pressure sensor are preferably all placed at a distance from the center C of the contact lens 1 sufficient for them to not disturb the eyesight of a user wearing the device of the invention, so that the device of the invention can be worn without significant disturbances and/or discomfort for the user over extended period of times, for example 10 hours, 24 hours or even some days, just like any usual contact lens.

The pressure sensor comprises an active strain gage 2, a passive strain gage 3, a rigid element 4 and a microprocessor 5.

The contact lens 1 is preferably a soft contact lens, made for example of a waterproof and/or silicone-based material, which adheres to the eyeball with a relatively high adhesion force. Variations of the intraocular pressure (IOP) generate deformations of the eyeball of a user. Typically, when the IOP rises, the eyeball dilates, and when the IOP diminishes, the eyeball contracts. When the device of the invention is worn by the user, the deformations of his or her eyeball induce deformations of the contact lens 1 that is in close contact with the eyeball, the amplitude of the deformations of the contact lens 1 being larger at its periphery.

The active strain gage 2 is configured and located on the contact lens 1 in order to be subjected to the deformations of the contact lens 1. According to the invention, a portion of the active strain gage 2 is placed around the center C of the contact lens 1 and at least partly encircles the center C. The active strain gage 2 thus describes, or covers, an arc of circle that is preferably centered on the center C of the contact lens 1.

The general shape of the portion of the active strain gage 2 that is placed around the center C is that of an arc of a circle. However, the configuration of this portion can vary within the frame of the invention, depending for example on the sought electrical properties of the active strain gage 2, the method used for its manufacturing, the place available on the contact lens, etc. The portion of the active strain gage 2 that is placed around the center C is for example made of one or more curved or circular segments forming one or more concentric arcs, or of one or more rectilinear segments forming for example one or more parts of a polygon, a mesh or any other adapted shape. A combination of one or more of the above shapes is also possible within the frame of the invention.

Independently of its configuration, the portion of the active strain gage 2 that is placed around the center C preferably covers an arc of at least 180° degrees around said center C, thus encircling the center C on at least 180°, i.e. on at least the half of its periphery, in order to provide for a sufficient and reliable sensing of the contact lens's deformations that are due to IOP variations, and thus in order to provide for a reliable measurement of IOP variations.

IOP variations induce deformations of the contact lens 1 worn by the user. The contact lens 1 is stretched when the IOP rises and contracted when it is reduced, or diminishes, thereby implying a variation of the contact lens's diameter. In order to reliably detect these diameter variations, the portion of the active gage 2 that is placed around the center C thus preferably covers an arc of at least 180°. This allows the active gage 2, independently of its local configuration, to detect diameter variations of the contact lens 1 rather than local deformations that could be due to local conditions that are not related to IOP variations.

Even more preferably, in order to maximize the length of the portion of the active strain gage 2 that is placed around the center C, thereby maximizing the sensitivity of the active strain gage 2, the portion of the active strain gage 2 that is placed around the center C covers as much as possible of an entire circle around the center C. According to a preferred embodiment of the invention, the portion of the active strain gage 2 that is placed around the center C thus preferably encircles the center C of the contact lens 1 on at least 270°, i.e. covering an arc of at least 270° degrees around said center C, a segment of the contact lens 1 being used by the essentially radial connections of the active strain gage 2 and of other elements of the pressure sensor to the microprocessor 5.

In a preferred embodiment, the active strain gage is a relatively thin and essentially circular electrical conductor placed at the periphery of the contact lens 1. Both ends of the active strain gage 2 are in electrical contact with the microprocessor 5. The section of the portion of the active strain gage 2 that is placed around the center C of the contact lens 1 is chosen small enough for the active stain gage to be deformable when submitted to the effects of the IOP variations. Preferably, the elasticity of the active strain gage 2 is equal or close to the elasticity of the contact lens 1. Even more preferably, the elasticity of the active strain gage 2 is equal to or higher than the elasticity of the contact lens 1. The active strain gage 2 is preferably made by etching, embossing and/or cutting of a thin metallic foil. In a variant embodiment, the active strain gage 2 is made of a thin metallic wire. In still a variant embodiment, the active strain gage is made by deposition of metal and/or of any other electrically conducting material, onto a preferably flexible and transparent substrate, for example onto a polyimide film 10.

According to the invention, and as explained above, the active strain gage 2 being united to the contact lens 1, deformations of the contact lens 1 induce deformations of the active strain gage 2, thereby modifying its physical properties, in particular its electrical properties. For example, if the IOP rises and the eyeball dilates, the contact lens 1 is extended at its periphery and the active strain gage 2 is stretched. This creates a diminution of the section of the portion of the active strain gage 2 placed around the center C of the contact lens 1 and thus an augmentation of its electrical resistance. By measuring the variations of the electrical resistance of the active strain gage 2 it is thus possible to detect and measure variations of the IOP.

The microprocessor 5 is programmed to measure the electrical resistance of the active strain gage 2 using methods known in the art.

Other factors than the deformation of the eyeball, and thereby of the contact lens 1, might however affect the electrical resistance of the active strain gage 2, in particular environmental parameters such as the temperature, the humidity, the ambient pressure, etc.

According to the invention, the pressure sensor of the invention comprises a passive strain gage 3 for measuring the effects of these other factors only, in particular for measuring the effects of the environmental parameters. According to the invention, the passive strain gage 3 is preferably similar in nature and configuration to the active strain gage 2, such that the effects of the environmental parameters on its physical properties are the same as or at least similar to the effects of these same parameters on the physical properties of the active strain gage 2. In particular, the passive strain gage 3 is preferably made of the same material and according to the same technology, or manufacturing process, as the active gage 2, and the shape and configuration of the passive strain gage 3 are preferably the same as or at least similar to the shape and configuration of the active gage 2. The passive strain gage 3 thus also comprises a portion placed around the center C of the contact lens 1 that preferably covers an arc of an angle close to the angle of the arc covered by the active gage 2.

According to the example illustrated in Fig. 1, the passive strain gage 3 is for example a thin and essentially circular electrical conductor placed around the center C of the contact lens 1. The passive strain gage 3 is preferably located closer to the center C of the contact lens 1 than the active strain gage 2. Both ends of the passive strain gage 3 are in electrical contact with the microprocessor 5.

Other configurations of the passive strain gage 3 are possible within the frame of the invention, the configuration of the passive strain gage 3 being preferably, but not necessarily, similar to that or the active gage 2. In particular, the portion of the passive strain gage 3 that is placed around the center C is for example made of one or more curved, or circular, segments forming one or more concentric arcs, or of one or more rectilinear segments forming for example one or more parts of a polygon, a mesh or any other adapted shape. A combination of one or more of the above shapes is also possible within the frame of the invention.

According to preferred embodiments of the invention, the passive strain gage 3 is of a configuration similar to that of the active gage 2, and preferably covers an arc with an angle close to the angle of the arc covered by the active strain gage 2, i.e. the passive strain gage 3 preferably encircles the center C of the contact lens 1 on an angle close to the angle with which the active gage 2 encircles the center C of the contact lens 1. The deformations induced in the passive strain gage 3 by possible variations of the environmental conditions are thereby similar to those induced by these same variations in the active gage 2. The effects of the variations of the environmental conditions on the electrical properties of the passive strain gage 3 are thus representative of the effects of the variations of the same environmental conditions on the electrical properties of the active gage 2.

The portion of the passive strain gage 3 that is placed around the center C of the contact lens 1 thus preferably covers an arc of at least 180°, i.e. encircles the center C of the contact lens on 180°. Even more preferably, the portion of the passive strain gage 3 that is placed around the center C covers an arc of at least 270° degrees around said center C, i.e. encircles the center C of the contact lens on at least 270°.

In order to avoid, or at least to minimize, any deformation of the passive strain gage 3 due to IOP variations, the pressure sensor of the invention further comprises a rigid element 4 having a portion located around the center C of the contact lens 1. The rigid element 4 is preferably sufficiently rigid for not being subjected to significant deformations when the user's eyeball is deformed. According to the invention, the portion of the passive strain gage 3 placed around the center C of the contact lens 1 is in immediate vicinity of the portion of the rigid element 4 placed around the center C of the contact lens 1. The portion of the passive strain gage 3 placed around the center C of the contact lens 1 is thus located in a region of the contact lens 1 that is rigidified by the rigid element 4 and is as such not, or only marginally, subjected to deformations due to IOP variations. The physical properties of the passive strain gage 3 are thus not noticeably modified when the eyeball is deformed because of IOP variations. Any noticeable change of the physical properties of the passive strain gage 3, in particular of its electrical resistance, can thus be considered as being due to other factors, in particular to variations of the environmental parameters.

The variations of the physical properties measured on the active strain gage 2 can thus be corrected by the variations of the physical properties measured on the passive strain gage 3 in order to determine the variations that are indeed essentially due to IOP variations. The variations of the intraocular pressure are thus determined for example on the basis of the result of the subtraction, from the measured variations of the electrical resistance of active strain gage 2, of the measured variations of the electrical resistance of passive strain gage 3, possibly multiplied or otherwise corrected by a calibration factor.

In the present description, the term "active strain gage", or "active gage", designates a strain gage of the pressure sensor of the device of the invention that is used for sensing deformations of the user's eyeball, and thus of the contact lens, that are due to variations of the user's IOP. The active strain gage is thus configured and placed on the device of the invention, in particular on the contact lens, in order to be as sensitive as possible to these deformations.

The term "passive strain gage", or "passive gage", however designates a strain gage of the pressure sensor of the device of the invention that is as insensitive as possible to the deformations of the eyeball that are due to variations of the user's IOP. Possible variations of the passive strain gage's physical properties are thus preferably due to variations of the environmental conditions only. The term "passive" thus refers to the fact that the strain gage only measures variations that are due to environmental conditions and is only marginally subjected to deformations due to the IOP variations.

According to the preferred embodiment illustrated in Fig. 1, the rigid element 4 is an antenna made for example of three concentric conductors placed around the center C of the contact lens 1, each made of a circular segment and each in electrical contact on both ends with the microprocessor 5. The antenna for example allows wirelessly transmitting signals between the microprocessor 5 and an external controller for measuring and recording the IOP variations over time. Preferably, the antenna 4 further allows providing electrical power to the microprocessor 5 through known induction powering methods.

According to an embodiment of the invention, when the microprocessor 5 is powered, the electrical resistance of both the active strain gage 2 and the passive strain gage 3 is measured, and possibly processed in the microprocessor 5 in order to determine an IOP value, either an absolute or a relative IOP value. The measured resistance values and/or the determined IOP value are then sent over the antenna 4 to the external controller for processing and/or for logging. Preferably, measurement cycles are initiated by the external controller and performed at regular intervals in order to allow a regular monitoring of the intraocular pressure. The frequency of the IOP measures depends on the needs, for example for diagnostic and/or experimental purposes, and is preferably determined by configuring the external controller.

In the preferred embodiment illustrated in Fig. 1, the rigid element 4 is an electrically conductive element paced around the center C and concentric with the passive strain gage 3 and the active strain gage 2. The configuration of the portion of the rigid element 4 that is located around the center C is preferably similar to that of the gages 2, 3, but with a significantly larger section, which makes it less elastic than the gages 2, 3 and thus preferably resistant to the deformations of the eyeball due to IOP variations. The rigid element 4 furthermore for example serves as an antenna for the pressure sensor to wirelessly communicate with an external controller.

The active strain gage 2 preferably lies along the periphery of the contact lens 1, where the amplitude of the deformations of the contact lens 1 due to IOP variations is the largest. The rigid element 4 and the passive strain gage 3 are preferably closer to the center C than the active strain gage 2, whilst not interfering with the eyesight of a user wearing the device of the invention. The rigid element 4 thereby rigidifies a central portion of the contact lens 1. Preferably, the passive strain gage 3 is situated in immediate vicinity of the rigid element 4, preferably along the inner side of the rigid element 4. In a variant embodiment, the passive strain gage 3 is at least partly situated between two concentric parts of the rigid element 4, for example between two rings of the antenna.

Other types of rigid elements are however possible within the frame of the invention in order to rigidify the part of the contact lens where the passive strain gage is located. In particular, the rigid element can have no other function than a mechanical one. The rigid element can for example be an element with a very low elasticity, placed in the immediate vicinity of the passive strain gage 3, or even attached to it, the rigid element being for example a relatively rigid substrate on which the passive strain gage 3 is affixed or grown through deposition, for example through metal vapour deposition. The rigid element is for example a rigid plastic, synthetic, metallic, or any other element with no other function than that of rigidifying a part of the contact lens in order to preserve the passive strain gage from deformations due to IOP variations when the device of the invention is worn by a user. According to a variant embodiment, the rigid element is for example a part, preferably in the form of a disc or a ring, of the contact lens 1, which is made more rigid than the rest of the contact lens, for example through a locally greater thickness and/or through the local use of another more rigid material than the material used for the rest of the preferably soft contact lens.

In the preferred embodiment illustrated in Fig. 1 and 2, the elements of the pressure sensor are assembled on a substrate 10, for example a polyimide film, and the pressure sensor is incorporated, or embedded, into the material forming the contact lens 1. In a variant embodiment, the pressure sensor, with or without substrate, is glued or otherwise affixed onto a side of the contact lens 1, preferably onto its external, convex, side.

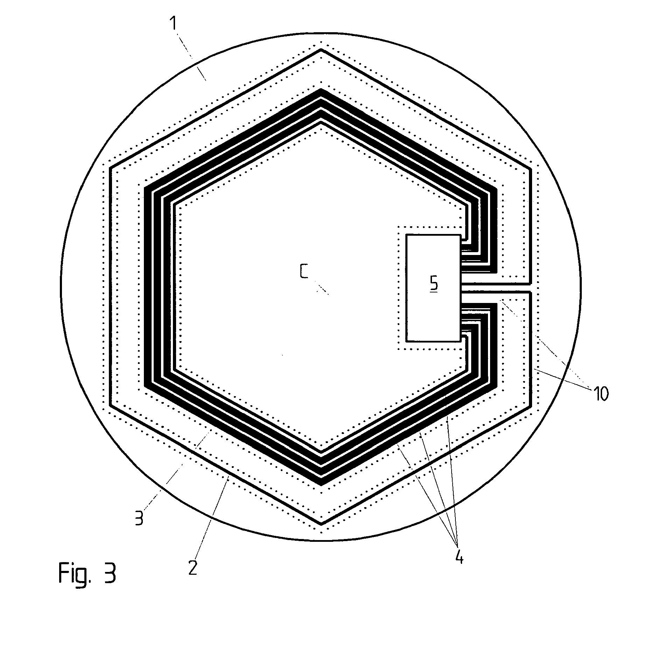

In the preferred embodiment illustrated in Fig. 1, the portions of the active strain gage 2, of the passive strain gage 3 and of the rigid element 4 that are located around the center C of the contact lens are essentially made of one or more circular segments. Other shapes are however possible within the frame of the invention. In particular, these elements can be essentially polygonal, for example a portion of a hexagon, an octagon or a dodecagon. Fig. 3 for example shows a variant embodiment of the device of the invention, wherein the portions of the active strain gage 2, of the passive strain gage 3 and of the rigid element 4 that are located around the center C of the contact lens are made of a plurality of rectilinear segments forming polygons. In this example, each of these portions is configured as one or more concentric polygons, in particular as one or more almost complete regular hexagons. The hexagons are only partly complete because a segment of the contact lens 1 accommodates the radial connections of the gages 2, 3 and of the rigid element 4 to the microprocessor 5.

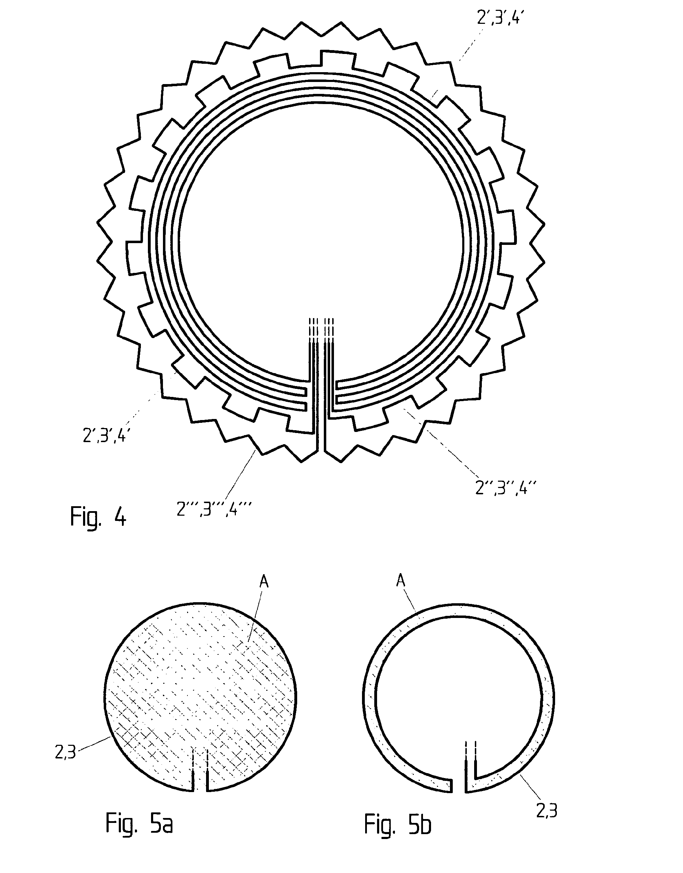

Fig. 4 shows further illustrative but not limiting examples of possible configurations, or shapes, for the portions of the active strain gage 2, of the passive strain gage 3 and/or of the rigid element 4, that are located around the center C.

Preferably, the active strain gage 2, the passive strain gage 3 are made of any sufficiently conductive material, such that variations of its resistance due to small deformations can still be reliably measured with usual techniques. Such material can be any conductive metal, alloy comprising one or more of these metals, polysilicon or semiconductor material. In a preferred embodiment, the active strain gage 2 and the passive strain gage 3 are made of platinum. The rigid element 4 is possibly made of the same material as the gages 2, 3, in particular if it also has an electrical function over the mechanical one. The substrate 10 is preferably made of a non-conductive material, for example polyimide, parylene or benzocyclobutene (BCB).

Preferably, the active strain gage 2 and the passive strain gage 3 have sections of 10 to 100 micrometers width and 100 to 500 nanometers thickness, more preferably of 10 to 20 micrometers width and 100 to 200 nanometers thickness. According to the embodiment illustrated in Fig. 1 and 2, each conductor of the rigid element 4 preferably has a section of 50 to 500 micrometers width and 1 to 50 micrometers thickness, more preferably of 150 to 250 micrometers width and 5 to 15 micrometers thickness. The thickness of the substrate 10, if any, is preferably between 1 and 500 micrometers, even more preferably between 5 and 10 micrometers. The use of other shapes, sections and/or thicknesses is however possible within the frame of this invention.

For the sake of readability and simplicity, the pressure monitoring device of the invention illustrated in Fig. 1 comprises one active strain gage 2 and one passive strain gage 3. It is however possible within the frame of the invention to provide a pressure sensor according to the invention with two or more active and/or passive strain gages. In particular, according to an advantageous configuration, the pressure sensor of the pressure monitoring device of the invention comprises two passive strain gages and two active strain gages that are interconnected in a Wheatstone bridge configuration, thereby allowing for a more efficient and reliable measurement of the IOP variations.

For the sake of readability and simplicity of the figures, the active strain gage 2 and the passive strain gage 3 are illustrated in their simplest form, i.e. their portion situated around the center C of the contact lens 1 is made of a single conductor that is electrically connected on both ends with the microprocessor 5. It is however possible, within the frame of the invention, to configure the portion of the passive and/or active gages situated around the center C with two or more concentric loops, each encircling the center C on an angle of at least 180°, preferably at least 270°.

This is illustrated in Fig. 5b by way of an illustrative but in no way limiting example of a correspondingly configured active or passive strain gage 2,3. In this example, the portion of the gage 2,3 that is configured for at least partly encircling the center of the contact lens is made of two concentric circular segments that are connected to each other on one side and are configured to be each connected to the microprocessor on the other side. In this example, the gage 2,3 thus comprises two concentric loops for at least partly encircling the center of the contact lens, and it is configured to be connected with both ends on the same side to the microprocessor 5. An advantage of this configuration is that, by increasing the number of loops of the portion of the gage 2,3 that is configured for at least partly encircling the center of the contact lens, the overall length of the strain gage 2,3 is increased, thereby increasing its sensitivity to mechanical deformations. Another advantage is that the area A defined by the electrical conductor of the gage 2,3 is significantly reduced in comparison to the area defined by the conductor of the gage illustrated for example in Fig. 5a, thereby reducing the sensitivity of the gage 2,3 of Fig. 5b to electromagnetic perturbations that could induce electrical currents in the gage 2,3 and thus perturb the measurement of the variations of its electrical properties due to mechanical deformations.

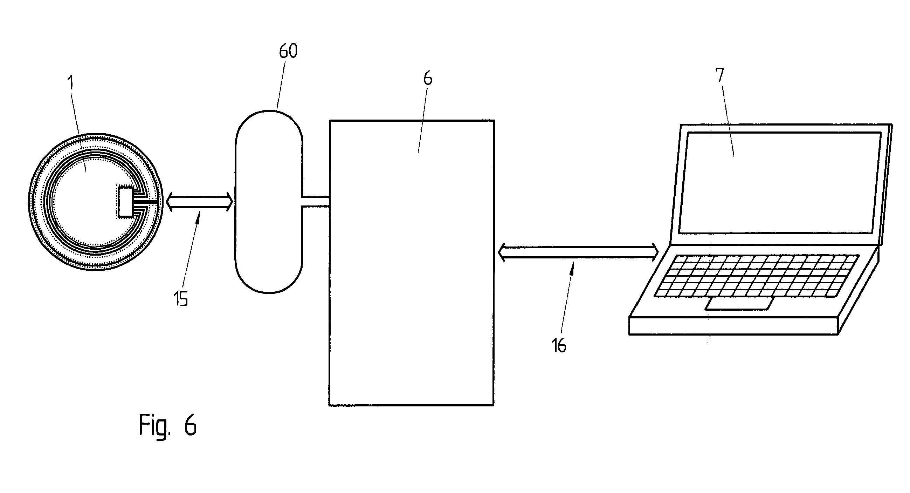

Fig. 6 is a schematic representation of a typical intraocular pressure monitoring system using the intraocular pressure monitoring device of the invention. According to the illustrated embodiment, the intraocular pressure monitoring system comprises the intraocular pressure monitoring device of the invention in the form of a contact lens 1 with a pressure sensor, a portable recording device 6 for communicating with the intraocular pressure monitoring device and storing the collected information during the IOP monitoring phases, and a computing device 7, for example a personal computer, for storing, analyzing, computing and/or displaying the data collected and stored by the portable communication device 6.

The portable recording device 6 comprises a first communication interface for communicating with the intraocular pressure monitoring device of the invention. The first communication interface is for example a wireless communication interface comprising an antenna 60 that is advantageously placed near the contact lens 1 when the intraocular pressure monitoring device of the invention is worn by a user. The antenna 60 is for example integrated into eyeglasses, not represented on the figures, and/or into a preferably disposable, flexible and hypoallergenic patch, also not represented on the figures, that are or is worn by the user during the IOP monitoring periods. Other means are however possible within the frame of the invention for placing the antenna 60 at a suitable distance from the intraocular pressure monitoring device of the invention when it is worn by a user. The portable recording device 6 further comprises a second communication interface for communicating with the computing device 7.

When monitoring IOP, the user wears the intraocular pressure monitoring device of the invention by placing the contact lens 1 on his or her eye, just like any ordinary contact lens, and carries the portable recording device 6, for example in a pocket or by hanging it around his or her neck. The antenna 60 is placed as close as possible to the user's eye wearing the contact lens 1 in order to allow the establishment of a first wireless communication channel 15 between the intraocular pressure monitoring device and the recording device 6. Preferably, the antenna 60 is furthermore oriented in a plane as parallel as possible to the plane of the antenna of the intraocular pressure monitoring device of the invention in order to allow for an efficient powering of the pressure sensor over the communication channel 15, which is for example a close distance inductive communication channel 15. The antenna is for example integrated in eyeglasses, and/or into a patch, preferably into a disposable, flexible and hypoallergenic patch, surrounding the eye, and/or in a cap or in another piece of clothing or accessory worn by the user. Preferably, the antenna 60 is centered with the antenna of the intraocular pressure monitoring device of the invention when the intraocular pressure monitoring device and the portable recording device 6 are worn by the user. The diameter of the antenna 60 of the portable recording device 6 is preferably larger than the diameter of the intraocular pressure monitoring device. The shape of the antenna 60 of the portable recording device 6 is for example round, oval, rectangular, or any other appropriate shape. The shape of the antenna 60 of the portable recording device 6 is preferably adapted to the shape of the device, for example the eyeglasses, the patch, the piece of garment, etc., to which it is attached.

According to a preferred embodiment, while monitoring IOP, the portable recording device 6 powers the intraocular pressure monitoring device through the first communication channel 15 at preferably regularly spaced time intervals and collects data sent by the microprocessor through the antenna of the intraocular pressure monitoring device. Collected data for example comprises electrical resistance values of the gages of the pressure sensor and/or a calculated IOP value. The collected data is stored in internal memory of the portable recording device 6. The intraocular pressure is for example measured at a frequency of 10 to 20 Hz during 10 to 60 seconds every 5 to 10 minutes. This allows a precise monitoring of the IOP variations over extended periods of time, including at night, while the user is asleep.

At some preferably predefined moments in time, for example once a day, once a week or once a month, the user and/or a practitioner connects the portable recording device 6 to a computing device 7, for example a personal computer, over a second, preferably wireless, communication channel 16, for example a Bluetooth communication channel. The second communication channel 16 can however also be a wired communication channel, for example a USB or any other appropriate communication channel. The data collected and stored in the internal memory of the portable recording device 6 is then transferred over the second communication channel 16 to the computing device 7 for further analysis and/or computing by the user and/or by the practitioner.

## Claims

1. Intraocular pressure monitoring device comprising a soft contact lens (1) and a pressure sensor united with said contact lens (1), said pressure sensor comprising:
- an active strain gage (2) for sensing deformations of a user's eyeball due to variations of the user's intraocular pressure,
- a passive strain gage (3) insensitive to the deformations of the eyeball due to variations of the user's intraocular pressure,
- a rigid element (4),
- a microprocessor,
said active strain gage (2), passive strain gage (3) and rigid element (4) being placed at a distance from the center (C) of the contact lens, said active strain gage (2) comprising a portion encircling said center (C) of the contact lens (1) on at least 180°,
wherein said passive strain gage (3) and said rigid element (4) each comprise a portion encircling said center (C) of the contact lens (1) on at least 180°, and said portion of said passive strain gage (3) situated around said center (C) of the contact lens (1) is placed in immediate vicinity of said portion of said rigid element (4) situated around said center (C) of the contact lens (1).

2. Intraocular pressure monitoring device according to the preceding claim, wherein said active strain gage (2) comprises a portion encircling said center (C) on at least 270°.

3. Intraocular pressure monitoring device according to one of the preceding claims, wherein said passive strain gage (3) and said rigid element (4) each comprise a portion encircling said center (C) on at least 270°.

4. Intraocular pressure monitoring device according to one of the preceding claims, wherein said portion of said passive strain gage (3) encircling said center (C) comprises a circular segment.

5. Intraocular pressure monitoring device according to one of claims 1 to 3, wherein said portion of said passive strain gage (3) encircling said center (C) comprises a plurality of rectilinear segments.

6. Intraocular pressure monitoring device according to the preceding claim, wherein said plurality of rectilinear segments forms a part of a regular polygon.

7. Intraocular pressure monitoring device according to one of the preceding claims, wherein said active strain gage (2), passive strain gage (3) and rigid element (4) are concentric.

8. Intraocular pressure monitoring device according to the preceding claim, wherein said passive strain gage (3) is closer than said active strain gage (2) to said center (C) of said contact lens (1).

9. Intraocular pressure monitoring device according to one of the preceding claims, wherein said rigid element (4) is an antenna allowing for wireless communications between said microprocessor (5) and an external device (6) and/or allowing for powering said microprocessor (5).

10. Intraocular pressure monitoring device according to one of claims 1 to 9, wherein said rigid element is a fibre or a synthetic element.

11. Kit comprising:
- a pressure monitoring device according to one of the preceding claims;
- a portable recording device (6) configured for communicating with said pressure monitoring device and for storing data received from said pressure monitoring device.

12. Kit according to the preceding claim, wherein said portable recording device (6) is configured for powering said pressure monitoring device over a wireless inductive communication channel (15).

13. Intraocular pressure monitoring system comprising:
- a pressure monitoring device according to one of the claims 1 to 10;
- a portable recording device (6) configured for communicating with said pressure monitoring device and for storing data received from said pressure monitoring device;
- a computing device (7) configured for communicating with said portable recording device (6) for receiving and/or processing and/or storing data received from said portable recording device (6).

14. Intraocular pressure monitoring system according to the preceding claim, wherein said portable recording device (6) is configured for powering said pressure monitoring device over a wireless inductive communication channel (15)

## Patentansprüche

1. Vorrichtung zur Überwachung des Augeninnendrucks mit einer weichen Kontaktlinse (1) und einem mit der besagten Kontaktlinse (1) fest verbundenen Drucksensor, wobei der besagte Drucksensor umfasst:
- einen aktiven Dehnungsmessstreifen (2) zum Abtasten von Verformungen am Augapfel eines Benutzers, die durch Variationen des Augeninnendrucks des Benutzers verursacht werden,
- einen passiven Dehnungsmessstreifen (3) unempfindlich auf die Verformungen am Augapfel, die durch Variationen des Augeninnendrucks des Benutzers verursacht werden,
- ein steifes Element (4),
- einen Mikroprozessor,
wobei der besagte aktive Dehnungsmessstreifen (2), der besagte passive Dehnungsmessstreifen (3) und das besagte steife Element (4) in einem Abstand zum Zentrum (C) der Kontaktlinse platziert werden, wobei der besagte aktive Dehnungsmessstreifen (2) einen das besagte Zentrum (C) der besagten Kontaktlinse (1) über mindestens 180° umschliessenden Anteil umfasst,
worin der besagte passive Dehnungsmessstreifen (3) und das besagte steife Element (4) jeweils einen das besagte Zentrum (C) der Kontaktlinse (1) über mindestens 180° umschliessenden Anteil umfassen, und worin der besagte, um das besagte Zentrum (C) der Kontaktlinse (1) gelegene Anteil des besagten passiven Dehnungsmessstreifens (3) in unmittelbarer Nachbarschaft des besagten Anteils des besagten, um das besagte Zentrum (C) der Kontaktlinse (1) gelegenen steifen Elements (4) platziert wird.

2. Vorrichtung zur Überwachung des Augeninnendrucks gemäss dem vorhergehenden Anspruch, worin der besagte aktive Dehnungsmessstreifen (2) einen das besagte Zentrum (C) über mindestens 270° umschliessenden Anteil umfasst.

3. Vorrichtung zur Überwachung des Augeninnendrucks gemäss einem der vorhergehenden Ansprüche, worin der besagte passive Dehnungsmessstreifen (3) und das besagte steife Element (4) jeweils einen das besagte Zentrum (C) über mindestens 270° umschliessenden Anteil umfassen.

4. Vorrichtung zur Überwachung des Augeninnendrucks gemäss einem der vorhergehenden Ansprüche, worin der besagte, das besagte Zentrum (C) umschliessende Anteil des besagten passiven Dehnungsmessstreifens (3) ein kreisförmiges Segment umfasst.

5. Vorrichtung zur Überwachung des Augeninnendrucks gemäss einem der Ansprüche 1 bis 3, worin der besagte Anteil des besagten, das besagte Zentrum (C) umschliessenden passiven Dehnungsmessstreifens (3) eine Vielzahl von geradlinigen Segmenten umfasst.

6. Vorrichtung zur Überwachung des Augeninnendrucks gemäss dem vorhergehenden Anspruch, worin die besagte Vielzahl von geradlinigen Segmenten einen Teil eines regelmässigen Vielecks bildet.

7. Vorrichtung zur Überwachung des Augeninnendrucks gemäss einem der vorhergehenden Ansprüche, worin der besagte aktive Dehnungsmessstreifen (2), der besagte passive Dehnungsmessstreifen (3) und das besagte steife Element (4) konzentrisch sind.

8. Vorrichtung zur Überwachung des Augeninnendrucks gemäss dem vorhergehenden Anspruch, worin der besagte passive Dehnungsmessstreifen (3) näher als der besagte aktive Dehnungsmessstreifen (2) am besagten Zentrum (C) der besagten Kontaktlinse (1) liegt.

9. Vorrichtung zur Überwachung des Augeninnendrucks gemäss einem der vorhergehenden Ansprüche, worin das besagte steife Element (4) eine Antenne ist, welche eine drahtlose Kommunikation zwischen dem besagten Mikroprozessor (5) und einer externen Vorrichtung (6) und/oder die Versorgung des besagten Mikroprozessors (5) ermöglicht.

10. Vorrichtung zur Überwachung des Augeninnendrucks gemäss einem der Ansprüche 1 bis 9, worin das besagte steife Element eine Faser oder ein synthetisches Element ist.

11. Kit mit:
- einer Vorrichtung zur Drucküberwachung gemäss einem der vorhergehenden Ansprüche;
- einer tragbaren Aufzeichnungsvorrichtung (6), so konfiguriert um mit der besagten Vorrichtung zur Drucküberwachung zu kommunizieren, und um Daten, die aus der besagten Vorrichtung zur Drucküberwachung empfangen werden, zu speichern.

12. Kit gemäss dem vorhergehenden Anspruch, worin die tragbare Aufzeichnungsvorrichtung (6) so konfiguriert ist, um die besagte Vorrichtung zur Drucküberwachung über einen drahtlosen induktiven Kommunikationskanal (15) zu versorgen.

13. System zur Überwachung des Augeninnendrucks mit:
- einer Vorrichtung zur Drucküberwachung gemäss einem der Ansprüche 1 bis 10,
- einer tragbaren Aufzeichnungsvorrichtung (6), so konfiguriert um mit der besagten Vorrichtung zur Drucküberwachung zu kommunizieren, und um Daten, die aus der besagten Vorrichtung zur Drucküberwachung empfangen werden, zu speichern
- einer Rechenvorrichtung (7), so konfiguriert um mit der besagten tragbaren Aufzeichnungsvorrichtung (6) zu kommunizieren, um aus der besagten tragbaren Aufzeichnungsvorrichtung (6) empfangene Daten zu empfangen und/oder zu verarbeiten und/oder zu speichern.

14. System zur Überwachung des Augeninnendrucks gemäss dem vorhergehenden Anspruch, worin die besagte tragbare Aufzeichnungsvorrichtung (6) so konfiguriert ist, um die besagte Vorrichtung zur Drucküberwachung über einen drahtlosen induktiven Kommunikationskanal (15) zu versorgen.

## Revendications

1. Dispositif de surveillance de la pression intraoculaire, comprenant une lentille cornéenne souple (1) et un capteur de pression solidaire de ladite lentille cornéenne (1), ledit dispositif de surveillance comprenant:
- une jauge active de contrainte (2) pour détecter les déformations du globe oculaire d'un utilisateur causées par des variations de la pression intraoculaire de l'utilisateur,
- une jauge passive de contrainte (3) insensible aux déformations du globe oculaire causées par des variations de la pression intraoculaire de l'utilisateur,
- un élément rigide (4),
- un microprocesseur,
ladite jauge active de contrainte (2), ladite jauge passive de contrainte (3) et ledit élément rigide (4) étant disposés à une distance du centre (C) de la lentille cornéenne, ladite jauge active de contrainte (2) comprenant une portion encerclant ledit centre (C) de la lentille cornéenne (1) sur au moins 180°,
dans lequel ladite jauge passive de contrainte (3) et ledit élément rigide (4) comprennent chacun une portion encerclant ledit centre (C) de la lentille cornéenne (1) sur au moins 180°, et dans lequel ladite portion de ladite jauge passive de contrainte (3) située autour dudit centre (C) de la lentille cornéenne (1) est placée dans le voisinage immédiat de ladite portion dudit élément rigide (4) située autour dudit centre (C) de ladite lentille cornéenne (1).

2. Dispositif de surveillance de la pression intraoculaire selon la revendication précédente, dans lequel ladite jauge active de contrainte (2) comprend une portion encerclant ledit centre (C) sur au moins 270°.

3. Dispositif de surveillance de la pression intraoculaire selon l'une des revendications précédentes, dans lequel ladite jauge passive de contrainte (3) et ledit élément rigide (4) comprennent chacun une portion encerclant ledit centre (C) sur au moins 270°.

4. Dispositif de surveillance de la pression intraoculaire selon l'une des revendications précédentes, dans lequel ladite portion de ladite jauge passive de contrainte (3) encerclant ledit centre (C) comprend un segment circulaire.

5. Dispositif de surveillance de la pression intraoculaire selon l'une des revendications 1 à 3, dans lequel ladite portion de ladite jauge passive de contrainte (3) encerclant ledit centre (C) comprend une pluralité de segments rectilinéaires.

6. Dispositif de surveillance de la pression intraoculaire selon la revendication précédente, dans lequel ladite pluralité de segments rectilinéaires forme une partie d'un polygone régulier.

7. Dispositif de surveillance de la pression intraoculaire selon l'une des revendications précédentes, dans lequel ladite jauge active de contrainte (2), ladite jauge passive de contrainte (3) et ledit élément rigide (4) sont concentriques.

8. Dispositif de surveillance de la pression intraoculaire selon la revendication précédente, dans lequel ladite jauge passive de contrainte (3) est plus proche dudit centre (C) de ladite lentille cornéenne (1) que ladite jauge active de contrainte (2).

9. Dispositif de surveillance de la pression intraoculaire selon l'une des revendications précédentes, dans lequel ledit élément rigide (4) est une antenne permettant des communications sans fil entre ledit microprocesseur (5) et un dispositif externe (6) et/ou permettant d'alimenter ledit microprocesseur (5).

10. Dispositif de surveillance de la pression intraoculaire selon l'une des revendications 1 à 9, dans lequel ledit élément rigide est une fibre ou un élément synthétique.

11. Kit comprenant:
- un dispositif de surveillance de la pression selon l'une des revendications précédentes;
- un dispositif portable d'enregistrement (6) configuré pour communiquer avec ledit dispositif de surveillance de la pression et pour stocker des données reçues à partir dudit dispositif de surveillance de la pression.

12. Kit selon la revendication précédente, dans lequel ledit dispositif portable d'enregistrement (6) est configuré pour alimenter ledit dispositif de surveillance de la pression par le biais d'un canal de communication inductif sans fil (15).

13. Système de surveillance de la pression intraoculaire, comprenant:
- un dispositif de surveillance de la pression intraoculaire selon l'une des revendications 1 à 10;
- un dispositif portable d'enregistrement (6) configuré pour communiquer avec ledit dispositif de surveillance de la pression et pour stocker des données reçues à partir dudit dispositif de surveillance de la pression;
- un dispositif informatique (7) configuré pour communiquer avec ledit dispositif portable d'enregistrement (6) pour recevoir et/ou traiter et/ou stocker des données reçues à partir dudit dispositif d'enregistrement portable (6).

14. Système de surveillance de la pression intraoculaire selon la revendication précédente, dans lequel ledit dispositif d'enregistrement portable (6) est configuré pour alimenter ledit dispositif de surveillance de la pression par le biais d'un canal de communication inductif sans fil (15).
